# EUROPEAN PATENT APPLICATION

(11) **EP 4 706 656 A1**
(43) Date of publication of application: **11.03.2026**
(21) Application number: 24797474.4
(22) Date of filing: 26.04.2024
(51) Int. Cl.: A61K 31/517, A61K 31/496, A61P 11/00, A61P 43/00

(54) **COMBINATION THERAPY OF DACOMITINIB AND NINTEDANIB FOR PREVENTION OR TREATMENT OF PULMONARY FIBROSIS**

(30) Priority: 28.04.2023 KR 20230056516
(71) Applicant: Korea Institute of Radiological & Medical Sciences, Seoul 01812 (KR)
(72) Inventor: LEE, Yoon-Jin, Seoul 07997 (KR); NAM, Jae Kyung, Seoul 01658 (KR); KIM, Jihee, Seoul 03763 (KR)
(74) Representative: Mewburn Ellis LLP
(86) International application number: PCT/KR2024/005690
(87) International publication number: WO 2024/225820

(57) **Abstract**

The present disclosure relates to a combination therapy of dacomitinib and nintedanib for the prevention or treatment of pulmonary fibrosis.

## Description

### [Technical Field]

The present disclosure relates to a combination therapy of dacomitinib and nintedanib for the prevention or treatment of pulmonary fibrosis.

### [Background Art]

Pulmonary fibrosis refers to a condition in which fibrous connective tissue proliferates in the lungs, destroying the normal lung structure and causing hardening and deterioration of the lung tissue.

Of the pulmonary fibrosis, idiopathic pulmonary fibrosis is a disease in which chronic inflammatory cells infiltrate the alveolar walls, causing various changes that harden the lungs, leading to severe structural changes in the lung tissue and a gradual deterioration in the lung function. Currently, no effective treatment is available.

Further, radiation therapy is often employed for patients with NSCLC who are ineligible for surgical resection. However, this frequently causes radiation-induced pulmonary fibrosis (RIPF).

To date, although Boehringer Ingelheim's Ofev having nintedanib as an active ingredient, which is a compound with a certain degree of efficacy, is known to slow the decline of pulmonary function, there has been a growing need to develop a more effective drug.

### [Disclosure]

### [Technical Problem]

The present inventors have made intensive efforts to develop a more effective drug for the prevention or treatment of pulmonary fibrosis, and as a result, they confirmed the prophylactic or therapeutic effects of a combination therapy of dacomitinib and nintedanib on pulmonary fibrosis, thereby completing the present disclosure.

### [Technical Solution]

An object of the present disclosure is to provide a therapy for the prevention, improvement, or treatment of pulmonary fibrosis, wherein dacomitinib or a pharmaceutically acceptable salt or solvate thereof; and nintedanib or a pharmaceutically acceptable salt or solvate thereof are administered in combination.

Another object of the present disclosure is to provide a pharmaceutical composition for preventing or treating pulmonary fibrosis, the pharmaceutical composition comprising dacomitinib or a pharmaceutically acceptable salt or solvate thereof, which is characterized in that the pharmaceutical composition is administered in combination with nintedanib or a pharmaceutically acceptable salt or solvate thereof.

Still another object of the present disclosure is to provide a pharmaceutical composition for preventing or treating pulmonary fibrosis, the pharmaceutical composition comprising nintedanib or a pharmaceutically acceptable salt or solvate thereof, which is characterized in that the pharmaceutical composition is administered in combination with dacomitinib or a pharmaceutically acceptable salt or solvate thereof.

Still another object of the present disclosure is to provide a combination comprising dacomitinib or a pharmaceutically acceptable salt or solvate thereof; and nintedanib or a pharmaceutically acceptable salt or solvate thereof.

Still another object of the present disclosure is to provide a pharmaceutical composition for preventing, improving, or treating pulmonary fibrosis, the pharmaceutical composition comprising the combination.

Still another object of the present disclosure is to provide a pharmaceutical kit for preventing, improving, or treating pulmonary fibrosis, the pharmaceutical kit comprising the combination.

Still another object of the present disclosure is to provide a method of preventing, improving, or treating pulmonary fibrosis, the method comprising the step of administering and/or applying the combination, pharmaceutical composition, or pharmaceutical kit to a subject in need thereof.

Still another object of the present disclosure is to provide a method of preventing, improving, or treating pulmonary fibrosis, the method comprising the step of administering and/or applying, to a subject in need thereof, a pharmaceutically effective amount of a composition comprising dacomitinib or a pharmaceutically acceptable salt or solvate thereof; and a composition comprising nintedanib or a pharmaceutically acceptable salt or solvate thereof in combination.

Still another object of the present disclosure is to provide use of the combination, pharmaceutical composition, or pharmaceutical kit in preventing, improving, or treating pulmonary fibrosis, and/or use thereof in preparing an agent for the prevention, improvement, or treatment of pulmonary fibrosis.

### [Advantageous Effects]

A combination therapy of the present disclosure has improved efficacy, as compared to a monotherapy, thereby being usefully applied for the prevention or treatment of pulmonary fibrosis.

### [Brief Description of the Drawings]

FIG. 1 shows micro-CT imaging for confirming the inhibitory effect on radiation-induced pulmonary fibrosis by administration of nintedanib or dacomitinib and co-administration of nintedanib and dacomitinib to mice with radiation-induced pulmonary fibrosis; and
FIG. 2 shows graphs of the degree of inflammation and the degree of fibrosis as determined by H&E and Masson's Trichrome staining after administration of nintedanib or dacomitinib and co-administration of nintedanib and dacomitinib to mice with radiation-induced pulmonary fibrosis.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a therapy for the prevention, improvement, or treatment of pulmonary fibrosis, wherein dacomitinib or a pharmaceutically acceptable salt or solvate thereof; and nintedanib or a pharmaceutically acceptable salt or solvate thereof are administered in combination.

Another aspect of the present disclosure provides a pharmaceutical composition for preventing or treating pulmonary fibrosis, the pharmaceutical composition comprising dacomitinib or a pharmaceutically acceptable salt or solvate thereof, which is characterized in that the pharmaceutical composition is administered in combination with nintedanib or a pharmaceutically acceptable salt or solvate thereof.

Still another aspect of the present disclosure provides a pharmaceutical composition for preventing or treating pulmonary fibrosis, the pharmaceutical composition comprising nintedanib or a pharmaceutically acceptable salt or solvate thereof, which is characterized in that the pharmaceutical composition is administered in combination with dacomitinib or a pharmaceutically acceptable salt or solvate thereof.

The dacomitinib of the present disclosure, also referred to as PF-00299804 or Vizimpro (trade name), is a commercially available compound and has a structure of following Chemical Formula 1.

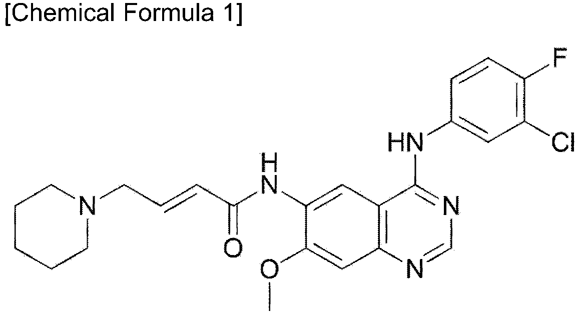

The IUPAC name for dacomitinib is (E)-N-(4-((3-chloro-4-fluorophenyl)amino)-7-methoxyquinazolin-6-yl)-4-(piperidin-1-yl)but-2-enamide.

Dacomitinib is a pan-human epidermal growth factor receptor (EGFR/HER1, HER2, HER4) inhibitor that is known to be used for EGFR mutations with exon 19 deletion or exon 21 L858R substitution.

The nintedanib of the present disclosure, also referred to as BIBF 1120 or Vargatef, Ofev (trade name), is a commercially available compound and has a structure of following Chemical Formula 2.

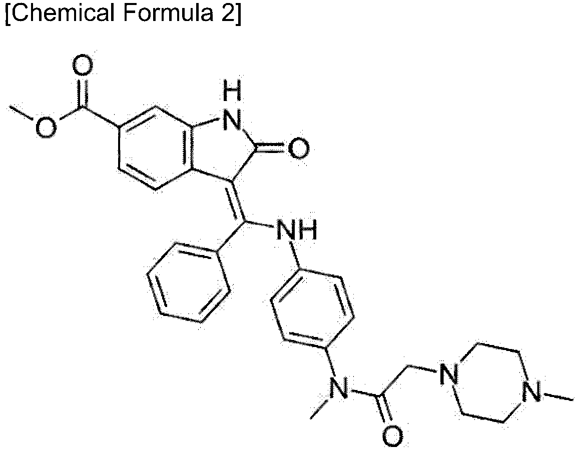

The IUPAC name for nintedanib is methyl (3Z)-3-{[(4-{methyl[(4-methylpiperazin-1-yl)acetyl]amino}phenyl)amino](phenyl)methylidene}-2-oxo-2,3-dihydro-1H-indole-6-carboxylate.

Meanwhile, the compounds of the present disclosure are intended to comprise not only compounds having a specific structural formula, but also clathrates, hydrates, solvates, or polymorphs thereof. In addition, the compounds of the present disclosure are intended to comprise pharmaceutically acceptable salts of the compounds of the present disclosure, unless the pharmaceutically acceptable salts thereof are mentioned. In one embodiment, the compounds of the present disclosure may exist as stereomerically pure compounds (e.g., substantially free of other stereoisomers (e.g., 85% ee or more, 90% ee or more, 95% ee or more, 97% ee or more, or 99% ee or more)), but are not limited thereto.

The term "hydrate" means the compound of the present disclosure or a pharmaceutically acceptable salt thereof that comprises a stoichiometric or non-stoichiometric amount of water bound by non-covalent intermolecular forces.

The term "clathrate" means the compound of the present disclosure or a pharmaceutically acceptable salt thereof in the form of a crystal lattice that comprises spaces (e.g., channels) having a guest molecule (e.g., a solvent or water) trapped within.

The phrase "pharmaceutically acceptable" or "pharmacologically acceptable" means being suitable for use as a pharmaceutical preparation, and may be employed herein to refer to compounds, materials, compositions, and/or dosage forms which are, within the scope of sound medical judgment, suitable for use in contact with the tissues of humans and animals without excessive toxicity, irritation, allergic response, or other problem or complication, commensurate with a reasonable benefit/risk ratio.

As used herein, the term "pharmaceutically acceptable salt" or "pharmacologically acceptable salt" refers to derivatives of the disclosed compounds, wherein a parent compound is modified by preparing acid or base salts thereof. Examples of pharmaceutically acceptable salts comprise, but are not limited to, mineral or organic acid salts of basic residues such as amines; alkali or organic salts of acidic residues such as carboxylic acids, etc. Examples of the pharmaceutically acceptable salts of the present disclosure may comprise, but are not limited to, salts commonly used in the pharmaceutical field, such as hydrochloride, hydrobromide, hydroiodide, hydrofluoride, sulfate, sulfonate, citrate, camphorate, malate, acetate, lactate, nicotinate, nitrate, succinate, phosphate, malonate, malate, salicylate, phenylacetate, stearate, formate, fumarate, urea, sodium, potassium, calcium, magnesium, zinc, lithium, cinnamate, methylamino, methanesulfonate, picrate, p-toluenesulfonate, naphthalenesulfonate, tartrate, triethylamino, dimethylamino and tri(hydroxymethyl)aminomethane.

The pharmaceutically acceptable salts of the present disclosure may be synthesized from parent compounds comprising a basic or acidic moiety by common chemical methods. Generally, such salts may be prepared by reacting free acid or base forms of the compounds with a sufficient amount of an appropriate base or acid in water or in an organic diluent such as ether, ethyl acetate, ethanol, isopropanol, or acetonitrile, or a mixture thereof.

As used herein, the term "solvate" or "pharmaceutically acceptable solvate" refers to a solvate formed from association of one or more solvent molecules and a compound. The term "solvate" comprises hydrates (e.g., hemi-hydrate, monohydrate, dihydrate, trihydrate, tetrahydrate, etc.).

The pharmaceutical composition according to the present disclosure may be prepared according to common methods in the pharmaceutical field. The pharmaceutical composition may be combined with an appropriate pharmaceutically acceptable carrier according to the dosage form, and as needed, may be prepared by further comprising excipients, diluents, dispersants, emulsifiers, buffers, stabilizers, binders, disintegrants, solvents, etc. The appropriate carrier, etc. may not interfere with the activity and properties of the compound according to the present disclosure, which may be selected differently according to the dosage form and formulation.

The pharmaceutical composition of the present disclosure may further comprise appropriate carriers, excipients, or diluents commonly used in the preparation of pharmaceutical compositions. The composition comprising a pharmaceutically acceptable carrier may be in various formulations of oral or parenteral forms. When formulated, they may be usually prepared using diluents or excipients such as fillers, extenders, binders, wetting agents, disintegrating agents, surfactants, etc., which are commonly used. Solid preparations for oral administration may comprise tablets, pills, powders, granules, capsules, etc., and these solid preparations are prepared by mixing one or more compounds with at least one excipient, for example, starch, calcium carbonate, sucrose or lactose, gelatin, etc. In addition to simple excipients, lubricants such as magnesium stearate, talc, etc. are also used. Liquid preparations for oral administration may comprise suspensions, internal solutions, emulsions, syrups, etc., and may comprise various excipients, for example, wetting agents, sweeteners, aromatics, preservatives, etc., in addition to simple diluents commonly used, such as water and liquid paraffin. Preparations for parenteral administration may comprise sterile aqueous solutions, non-aqueous solvents, suspensions, emulsions, lyophilized preparations, and suppositories. Propylene glycol, polyethylene glycol, vegetable oils such as olive oil, and injectable esters such as ethyl oleate may be used as non-aqueous solvents and suspensions. Witepsol, Macrogol, Tween 61, cacao butter, laurin butter, glycerogelatin, etc., may be used as a base for the suppository.

Further, the pharmaceutical composition of the present disclosure may have any one dosage form selected from the group consisting of, but is not limited to, tablets, pills, powders, granules, capsules, suspensions, internal solutions, emulsions, syrups, sterile aqueous solutions, non-aqueous solvents, suspensions, emulsions, lyophilized preparations, and suppositories.

Each component of the pharmaceutical composition according to the present disclosure may be comprised in a pharmaceutically effective amount in the pharmaceutical composition.

The "pharmaceutically effective amount" means an amount that is sufficient to inhibit or alleviate increased vascular permeability at a reasonable benefit/risk ratio applicable to medical treatment. The effective dose level may be determined according to factors comprising a subject' type and severity, age, sex, activity of the drug, sensitivity to the drug, time of administration, route of administration and excretion rate, duration of treatment, drugs used concurrently, and other factors well known in the medical field.

The effective dose level of the pharmaceutical composition may be determined according to factors comprising the purpose of use, the patient's age, sex, body weight, and health conditions, the type of disease, severity, activity of the drug, sensitivity to the drug, method of administration, time of administration, route of administration and excretion rate, duration of treatment, drugs used in combination or concurrently, and other factors well known in the medical field. For example, although not constant, it may be generally administered at a dose of 0.001 mg/kg to 100 mg/kg, for example, 0.01 mg/kg to 10 mg/kg once or several times a day. The above dosages do not limit the scope of the present disclosure in any aspect.

The pharmaceutical composition may be appropriately administered to a subject according to a common method, route of administration, and dosage used in the art, depending on the purpose or need. Examples of the route of administration may comprise oral, parenteral, subcutaneous, intraperitoneal, intrapulmonary, and intranasal administration, and parenteral injection comprises intramuscular, intravenous, intraarterial, intraperitoneal, or subcutaneous administration. In addition, an appropriate dosage and frequency of administration may be selected according to a method known in the art, and the amount and frequency of administration of the pharmaceutical composition of the present disclosure to be actually administered may be appropriately determined by various factors, such as the type of symptom to be treated, route of administration, sex, health conditions, diet, a subject's age and body weight, and severity of the disease.

The term "administering" means introducing the pharmaceutical composition of the present disclosure to a subject by any suitable method, and as for the route of administration, the composition may be administered via various routes comprising oral or parenteral routes as long as it reaches a target tissue.

The pharmaceutical composition may be administered to any animal which may develop pulmonary fibrosis, and the animal may comprise, for example, humans and primates, as well as livestock such as cows, pigs, horses, dogs, etc. In some embodiments, the animal may be an animal other than a human.

The pharmaceutical composition may be administered by an appropriate route of administration according to the formulation form, and may be administered via various routes, either oral or parenteral route, as long as it may reach a desired tissue. The method of administration may be made via common methods without particular limitation, for example, oral, rectal or intravenous, intramuscular, skin application, respiratory inhalation, endometrial, or intracerebroventricular injection, etc.

In the present disclosure, "combined administration", "administered in combination", or "administering in combination" means not only simultaneous administration, but also an administration form in which dacomitinib or a pharmaceutically acceptable salt or solvate thereof and nintedanib or a pharmaceutically acceptable salt or solvate thereof both act together on a subject so that each substance may perform a function at a level equal to or higher than its original function. Accordingly, when the term "in combination" is used herein, this is to be understood that administration is performed simultaneously, individually, sequentially, or in reverse order, and the order is unlimited. When the administration is performed sequentially, in reverse order, or individually, the order of administration is not particularly limited, but the administration interval of the second ingredients is to be such that the beneficial effects of the combination are not lost.

In the present disclosure, (i) the composition comprising dacomitinib or a pharmaceutically acceptable salt or solvate thereof, and (ii) the composition comprising nintedanib or a pharmaceutically acceptable salt or solvate thereof may be administered as follows, but are not limited thereto:
a) (i) administering dacomitinib or the pharmaceutically acceptable salt or solvate thereof; and (ii) nintedanib or the pharmaceutically acceptable salt or solvate thereof in the form of a mixture; or
b) (i) administering dacomitinib or the pharmaceutically acceptable salt or solvate thereof; and (ii) nintedanib or the pharmaceutically acceptable salt or solvate thereof in the form of separate preparations, but are not limited thereto.

When (i) dacomitinib or the pharmaceutically acceptable salt or solvate thereof; and (ii) nintedanib or the pharmaceutically acceptable salt or solvate thereof are in separate preparations, (i) and (ii) may each be formulated in separate preparations, and these formulations may be administered simultaneously, individually, sequentially, or in reverse order.

The therapeutically effective dosage of each active ingredient when administered in combination may vary depending on the specific compound or pharmaceutical composition employed, the mode of administration, symptoms to be treated, severity of the symptoms to be treated, the species, body weight, sex, diet, and age of the warm-blooded animal. Therefore, the dosage regimen using the compounds of the present disclosure is selected in accordance with a variety of factors comprising the route of administration and the renal and hepatic function of the patient. A physician, clinician, or veterinarian of ordinary skill may readily determine and prescribe the effective amount of the drug required to prevent, counter, or arrest the progress of the symptoms. Optimal precision in achieving concentration of the drug within the range that yields efficacy without toxicity requires a regimen based on the kinetics of the drug's availability to target sites. This involves a consideration of the distribution, equilibrium, and elimination of the drug. Therefore, the dosage regimen, i.e., the dosage level and frequency of administration of any individual component of the present disclosure, may be adjusted to provide the optimal therapeutic response.

Still another aspect of the present disclosure provides a combination comprising dacomitinib or a pharmaceutically acceptable salt or solvate thereof; and nintedanib or a pharmaceutically acceptable salt or solvate thereof.

As used herein, the term "combination" refers to those having use of the combined administration of dacomitinib or the pharmaceutically acceptable salt or solvate thereof; and nintedanib or the pharmaceutically acceptable salt or solvate thereof, and may be understood to have the same meaning as combined use. This also comprises, but is not limited to, the forms of a pharmaceutical composition and a pharmaceutical kit, which is characterized by the combined use of dacomitinib or the pharmaceutically acceptable salt or solvate thereof; and nintedanib or the pharmaceutically acceptable salt or solvate thereof.

In the present disclosure, the "kit" may comprise the combination or composition according to the present disclosure for the combined administration of dacomitinib or the pharmaceutically acceptable salt or solvate thereof; and nintedanib or the pharmaceutically acceptable salt or solvate thereof. Specifically, the kit of the present disclosure may comprise dacomitinib or the pharmaceutically acceptable salt or solvate thereof; and nintedanib or the pharmaceutically acceptable salt or solvate thereof, which are formulated as a single preparation, or dacomitinib or the pharmaceutically acceptable salt or solvate thereof; and nintedanib or the pharmaceutically acceptable salt or solvate thereof, which are separate preparations. The kit may further comprise substances required for the combined administration of the two substances, but is not necessarily limited thereto.

The term "pulmonary fibrosis" or "lung fibrosis" refers to a respiratory disease in which the lung tissue becomes hard, causing serious breathing difficulties. Pulmonary fibrosis may be caused by various factors, comprising radiation, tuberculosis, syphilis, pneumoconiosis, or viral infection, etc., and may also be caused by unknown causes, all of which fall within the scope of pulmonary fibrosis of the present disclosure.

In one embodiment of the present disclosure, pulmonary fibrosis may be selected from radiation-induced pulmonary fibrosis, acute pulmonary fibrosis, and idiopathic pulmonary fibrosis.

The "idiopathic lung fibrosis" or "idiopathic pulmonary fibrosis (IPF) of the present disclosure refers to an interstitial lung disease of unknown cause in which repetitive inflammation due to alveolar damage induces fibrosis, leading to respiratory failure in the patient.

In one embodiment of the present disclosure, pulmonary fibrosis may be a side effect of radiotherapy resulting from exposure of the normal tissue during radiotherapy for cancer, or a side effect of chemotherapy for anticancer treatment.

The radiotherapy or chemotherapy for cancer that may induce pulmonary fibrosis may comprise, but is not limited to, treatment for breast cancer, lung cancer, bone cancer, pancreatic cancer, skin cancer, oral cancer, oropharyngeal cancer, uterine cancer, ovarian cancer, rectal cancer, stomach cancer, endometrial carcinoma, cervical carcinoma, vaginal carcinoma, small intestine cancer, thyroid cancer, parathyroid cancer, prostate cancer, chronic or acute leukemia, lymphocytic lymphoma, bladder cancer, kidney cancer, liver cancer, colon cancer, or brain tumors.

In one embodiment, the pharmaceutical composition of the present disclosure may be administered before or after radiation exposure.

The term "treatment" means all of the actions by which the symptoms of fibrosis have taken a turn for the better or been modified favorably by the administration of the pharmaceutical composition, and the "prevention" means all of the actions by which the occurrence of fibrosis is restrained or retarded by the administration of the pharmaceutical composition.

As used herein, the term "improvement" means all of the actions that at least diminish a parameter associated with pulmonary fibrosis by the administration of the composition of the present disclosure, for example, the severity of symptom.

Still another aspect of the present disclosure provides a method of preventing, improving, or treating pulmonary fibrosis, the method comprising the step of administering and/or applying the combination, pharmaceutical composition, or pharmaceutical kit, each comprising dacomitinib or a pharmaceutically acceptable salt or solvate thereof; and nintedanib or a pharmaceutically acceptable salt or solvate thereof, to a subject in need thereof.

Still another aspect of the present disclosure provides a method of preventing, improving, or treating pulmonary fibrosis, the method comprising the step of administering and/or applying, to a subject in need thereof, a pharmaceutically effective amount of a composition comprising dacomitinib or a pharmaceutically acceptable salt or solvate thereof; and a composition comprising nintedanib or a pharmaceutically acceptable salt or solvate thereof in combination.

Still another aspect of the present disclosure provides use of the composition comprising dacomitinib in a pharmaceutically effective amount or a pharmaceutically acceptable salt or solvate thereof; and a pharmaceutical composition comprising nintedanib or a pharmaceutically acceptable salt or solvate thereof, or the pharmaceutical kit in preventing, improving, or treating pulmonary fibrosis, and/or use thereof in preparing an agent for the prevention, improvement, or treatment of pulmonary fibrosis.

The composition comprising dacomitinib or the pharmaceutically acceptable salt or solvate thereof; and nintedanib or the pharmaceutically acceptable salt or solvate thereof, pulmonary fibrosis, preventing and treating are as described in other aspects.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1: Confirmation of Inhibitory Effect on Radiation-Induced Pulmonary Fibrosis

Seven-week-old male C57BL/6 mice were divided into groups receiving nintedanib, which is an FDA-approved pulmonary fibrosis inhibitor, dacomitinib, which is an EGFR TKI, and a combination thereof, and orally administered one hour prior to stereotactic body radiation therapy (SBRT) (90 Gy, 4 mm collimator). Two weeks after radiation, the degree of pulmonary fibrosis and the inhibitory effect on radiation-induced lung injury were observed.

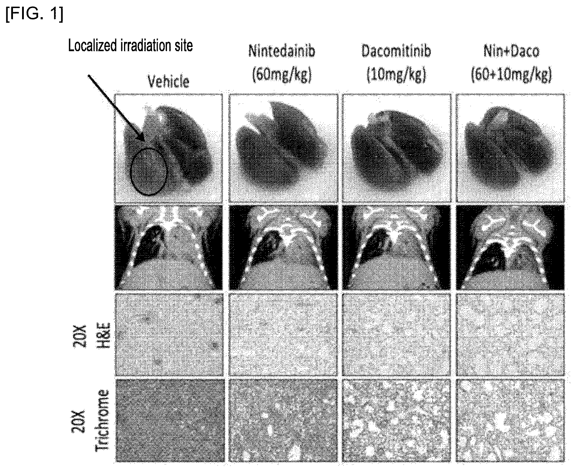

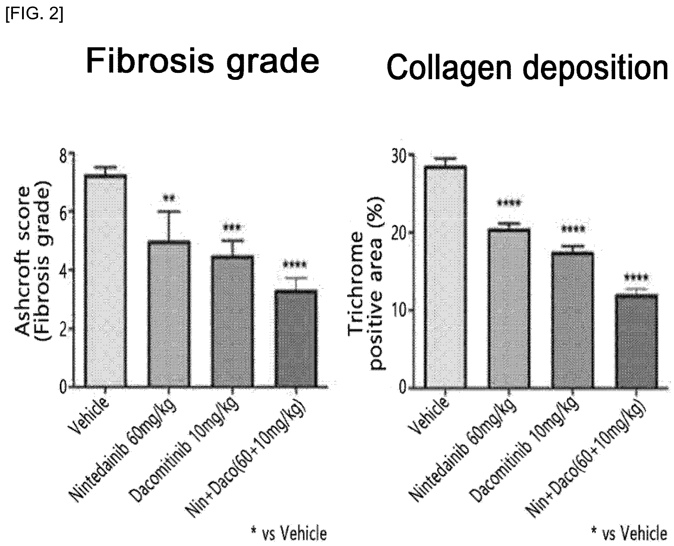

The nintedanib group received 60 mg/kg daily, the dacomitinib group received 10 mg/kg daily, and the combination group received 60 mg/kg of nintedanib and 10 mg/kg of dacomitinib. All drugs were orally administered daily. The results of micro-CT scanning taken two weeks after radiation are shown in FIG. 1. In addition, to analyze the degree of inflammatory response and the degree of fibrosis, H&E staining and Masson's Trichrome staining were performed and the grade of pulmonary fibrosis and the degree of collagen deposition are graphically represented in FIG. 2.

As a result of the experiment, the untreated group showed an increase in the degree of fibrosis two weeks after radiation exposure. As compared to the untreated group, the nintedanib and dacomitinib-treated groups showed reduced fibrosis, respectively, but the group that received the two drugs in combination showed a significant decrease in pulmonary fibrosis.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A pharmaceutical composition for preventing or treating pulmonary fibrosis, the pharmaceutical composition comprising dacomitinib in a pharmaceutically effective amount or a pharmaceutically acceptable salt or solvate thereof,
wherein the pharmaceutical composition is administered in combination with nintedanib or a pharmaceutically acceptable salt or solvate thereof.

2. The pharmaceutical composition of claim 1, wherein the dacomitinib is a compound represented by following Chemical Formula 1:

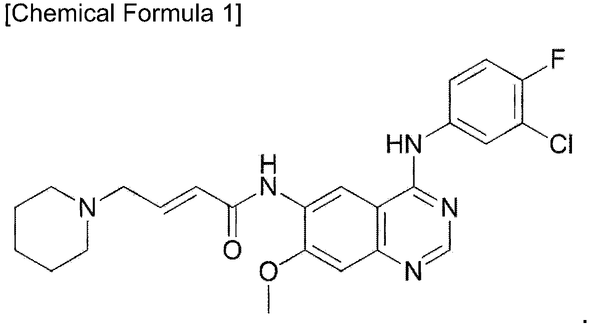

3. The pharmaceutical composition of claim 1, wherein the nintedanib is a compound represented by following Chemical Formula 2:

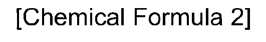

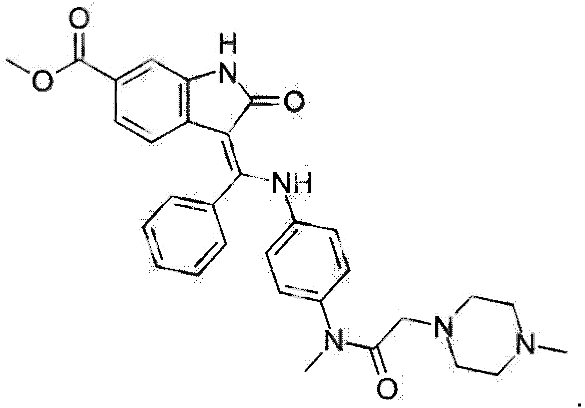

4. The pharmaceutical composition of claim 1, wherein the pharmaceutical composition further comprises a pharmaceutically acceptable carrier, excipient, or diluent.

5. The pharmaceutical composition of claim 1, wherein the dacomitinib or the pharmaceutically acceptable salt or solvate thereof is administered with the nintedanib or the pharmaceutically acceptable salt or solvate thereof simultaneously, sequentially, or in reverse order.
